# EUROPEAN PATENT APPLICATION

(11) **EP 4 461 299 A1**
(43) Date of publication of application: **13.11.2024**
(21) Application number: 23172137.4
(22) Date of filing: 08.05.2023
(51) Int. Cl.: A61K 31/713, A61K 38/48, A61K 45/06, A61P 17/08, A61P 17/12

(54) **TOPICAL COMPOSITIONS FOR THE TREATMENT OF SEBORRHEIC KERATOSES**

(71) Applicant: Martin, Brigitte und Theo Deichmann sebolysis GbR, 63571 Gelnhausen (DE)
(72) Inventor: DEICHMANN, Martin, 63571 Gelnhausen (DE)
(74) Representative: Fuchs Patentanwälte Partnerschaft mbB

(57) **Abstract**

A topical composition is presented comprising a therapeutically effective amount of an active agent which degrades keratin 1 (KRT1) and/or keratin 10 (KRT 10) for use in the treatment of seborrheic keratoses (very common, benign, non-infectious, ugly warts on skin) (figure 7).

## Description

### Technical Field

The present invention generally relates to novel topical compositions for the treatment of seborrheic keratoses. The topical compositions and uses thereof allow a gentle and effective treatment.

### Background

Seborrheic keratoses are very common skin growths that are typically benign (non-cancerous). They can appear in various colours such as black, brown, or light tan, and can range in size from a few millimetres to several centimetres. They often have a waxy, scaly, or rough texture and may have a "stuck-on" appearance, as if they are attached to the skin.

Seborrheic keratoses are most commonly found on the face, chest, shoulders, back, or other areas of the body. They are more common in older individuals and can run in families. It is estimated that up to 83% of people over the age of 50 have at least one seborrheic keratosis, and they become even more common as people age. This means that for example in Germany alone about 25 million people exist which do have at least one seborrheic keratosis. Seborrheic keratoses are also more common in people with a family history of the condition. While they can occur in people of all skin types, they are more common in people with fair skin.

Seborrheic keratoses are usually harmless and do not require treatment, but many people may choose to have them removed for cosmetic reasons or if the growths are irritated or itchy. There are several methods for removing seborrheic keratoses, including:
- Cryotherapy: This involves freezing the growth with liquid nitrogen, causing it to fall off within a few weeks.
- Curettage: This involves scraping the growth with a sharp instrument.
- Electrosurgery: This involves using a high-frequency electrical current to burn off the growth.
- Laser surgery: This involves using a laser to vaporize the growth.
- Topical medications: Certain medications may be applied topically to remove the outer layers of the growth, such as salicylic acid, lactic acid, and urea. To date, no effective commercial topical treatment is available to remove seborrheic keratoses.

While current treatments for seborrheic keratoses can be effective, they also have potential disadvantages and risks to consider. Some of these include:
- Cryotherapy, curettage, electrosurgery, and laser can leave scars, especially if the growth is large or in a visible area.
- Topical medications may cause skin irritation, redness, and flaking, and may take several weeks to months to show results.
- Laser surgery is expensive and usually not covered by insurance.
- In rare cases, the removal of a seborrheic keratosis can cause bleeding, infection, or change in skin pigmentation.

Thus, there is a need in the field to develop easy-to-use and patient-friendly alternatives, which may even allow the treatment of seborrheic keratoses by self-medication. These alternatives are disclosed in the present application.

### Details of the Invention

In a first aspect the disclosure pertains to a topical composition comprising a therapeutically effective amount of an active agent which suppresses keratin 1 (KRT1) and/or keratin 10 (KRT 10) activity for use in the treatment of seborrheic keratoses.

In another embodiment the disclosure pertains to a topical composition comprising a therapeutically effective amount of an active agent which suppresses keratin 1 (KRT1) and/or keratin 10 (KRT 10) activity in one embodiment in skin epidermis for use in the treatment of seborrheic keratoses.

In yet another embodiment the disclosure pertains to a topical composition comprising a therapeutically effective amount of an active agent which degradates keratin 1 (KRT1) and/or keratin 10 (KRT 10) in skin epidermis for use in the treatment of seborrheic keratoses.

A "topical composition" is defined hereinunder as a medication or cosmetic product that is designed to be applied to the skin. It can take various forms such as creams, lotions, gels, ointments, solutions, sprays, foams, powders, patches, and transdermal systems. Topical compositions are used for local or regional effects, meaning they act on the area where they are applied, rather than systemic effects throughout the body. They may contain one or more active ingredients that are intended to treat a variety of skin conditions or provide cosmetic benefits, such as moisturizing or sun protection. Topical compositions are formulated to optimize absorption, stability, and effectiveness while minimizing irritation, toxicity, and other adverse effects.

A "therapeutically effective amount" is the minimum dose or concentration of a drug or other therapeutically agent that produces the desired therapeutically effect in a patient. This amount varies depending on the particular drug, the patient's condition, and other factors such as age, weight, and overall health. The therapeutically effective amount is typically determined through clinical trials and is based on the drug's pharmacological properties and the intended therapeutic outcome. The goal of using a therapeutically effective amount is to achieve the desired therapeutically effect while minimizing the risk of adverse effects or toxicity.

In one embodiment the topical composition comprises a therapeutically effective amount of the active agent sufficient to supresss keratin 1 (KRT-1) and/or keratin 10 (KRT-10) activity, in one embodiment sufficient to degrade keratin 1 (KRT-1) and/or keratin 10 (KRT-10) in the skin epidermis within the treatment interval. An therapeutically effective amount of the agent is achieved if its inhibitive and/or degradative activity at 37°C and pH 7 is at least 10 nkat (nano katal), in one embodiment at least 25 nkat, more in one embodiment at least 50 nkat, even more in one embodiment at least 100 nkat, most in one embodiment at least 250 nkat and more; and up to 1kat or less, in one embodiment up to 1*10⁻¹ kat or less, more in one embodiment up to 1 *10⁻² kat or less, even more in one embodiment up to 1*10⁻³ kat or less, even more in one embodiment up to 1*10⁻⁴ kat or less, most in one embodiment up to 1 *10⁻⁵ kat or less, in the topical composition. Wherein 1 katal (kat) = 1 mol substrate s⁻¹. In one embodiment the catalytic activity at 37°C and pH 7 is at least about 85*10⁻⁹ kat and up to 1.2*10⁻³ kat. The higher the catalytic activity the better, however, the ideal catalytic activity may be chosen depending on the application of the composition. For example, the catalytic activity of a cream used for short term treatment, e.g., for a facemask, between 5 minutes and up to less than 30 minutes, between 15 minutes and up to less than 2 hours, may necessitate a higher catalytic activity of at least 1*10⁻² kat, whereas the composition used for a skin patch which is worn at least 12 hours, at least 24 hours 48 hours. In some embodiments worn up to 3 days, up to 4 days, or up to 1 week (168 hours), may only necessitate a catalytic activity between 1*10⁻⁸ kat and 1*10⁻² kat.

A "nano katal" is defined herein as a unit of measurement used in biochemistry and enzymology to express the catalytic activity of enzymes. One nano-katal is equal to one billionth (10⁻⁹) of a katal, which is the amount of enzyme activity required to convert one mole of substrate per second under specified conditions. It is commonly used to express low levels of enzyme activity, particularly in research and medical contexts.

In yet another embodiment the therapeutically effective amount (wt/vol) of active ingredient, e.g., a serralysin family metalloprotease and/or a hydrolase, in the final product may be at least 0.25 µg/ml, at least 0.5 µg/ml, at least 0.75 µg/ml, at least 1 µg/ml, at least 1.5 µg/ml, at least 2 µg/ml, at least 2.5 µg/ml. In yet another embodiment the amount of active ingredient in the final product may be up to 3 µg/ml, up to 4 µg/ml, up to 5 µg/ml, up to 6 µg/ml, up to 7 µg/ml, up to 8 µg/ml, up to 9 µg/ml, up to 10 µg/ml. In yet another embodiment the amount of active ingredient may be between 0.25 µg/ml and 10 µg/ml, between 0.5 µg/ml and 9 µg/ml, between 0.75 µg/ml and 8 µg/ml, between 1 µg/ml and 7 µg/ml, between 2 µg/ml and 6 µg/ml, between 2.5 µg/ml and 5 µg/ml.

In yet another embodiment the therapeutically effective amount of active ingredient, e.g., a serralysin family metalloprotease and/or a hydrolase, in the final product depends on the keratin, type I/cytoskeletal 10 and/or keratin, type II/cytoskeletal 1-content in the target keratosis. For example, a ratio of active ingredient to keratin, type I/cytoskeletal 10 and keratin, type II/cytoskeletal 1-content of about 5:1 to about 1:5 may be present. In one embodiment a ratio of active ingredient to either keratin, type I/cytoskeletal 10 and/or keratin, type II/cytoskeletal 1-content about 5:1, about 4:1, about 3:1, about 2:1, about 1:1 is present. In yet another embodiment a ratio of active ingredient to either keratin, type I/cytoskeletal 10 and/or keratin, type II/cytoskeletal 1-content about 1:2, about 1:3, about 1:4, about 1:5 is present.

In yet another embodiment the therapeutically effective amount of active ingredient is achieved if it passes the KRT1/KRT10-lysis test and/or lytic seborrheic keratosis cell cluster test with a score of at least 3.

The term "suppresses" or "suppresses keratin 1 and/or keratin 10 activity" as used herein refers to any reduction of keratin 1 and/or keratin 10 function in the target, i.e., skin epidermis, by the active agent, i.e. a reduction of protein activity to 50% as compared to target, i.e., skin epidermis, which was not treated with the active agent, in further embodiments a reduction to 40%, or a reduction to 30%, or a reduction to 20%, or a reduction to 10%, or a reduction to 0% (i.e., absence of keratin 1 and/or keratin 10 function in the target). The term "suppresses" or "suppresses keratin 1 and/or keratin 10 activity" comprises any reduction of keratin 1 and/or keratin 10 function selected from the inhibition of protein expression of keratin 1 and/or keratin 10, the inhibition of the protein activity of keratin 1 and/or keratin 10, and the degradation of keratin 1 and/or keratin 10, as well as any combination thereof.
- Wherein the term "inhibition of protein expression of keratin 1 and/or keratin 10" refers to an active substance which specifically reduces or completely prevents the protein expression of keratin 1 and keratin 10 in the target skin cell. inhibitors of protein expression are normally molecules or compounds that interfere with the process of protein synthesis, either by preventing the transcription of DNA into mRNA or by blocking the translation of mRNA into protein. Some examples of inhibitors of protein expression include:
   1. Transcription inhibitors: These are molecules that prevent RNA polymerase from transcribing DNA into mRNA.
   2. Translation inhibitors: These are molecules that interfere with the translation of mRNA into protein.
   3. Post-translational inhibitors: These are molecules that prevent the processing of proteins after they have been translated.
   Examples for such specific inhibitors of protein expression may be siRNA (short interfering RNA) designed to specifically bind to keratin 1 and/or keratin 10 mRNA, thereby utilizing the RNA interference (RNAi)mechanism. In addition, there are some drugs that indirectly affect keratin expression or function. For example, retinoids, which are derivatives of vitamin A, have been shown to modulate keratinocyte differentiation and induce the expression of keratin 1 and keratin 10 in the skin. Other drugs, such as methotrexate, have been shown to decrease keratinocyte proliferation and differentiation, and may indirectly affect keratin expression.
   Also, some siRNAs are reported which indirectly inhibit the protein expression of keratin 1 and/or keratin 10, e.g., by interfering with upstream proteins and/or keratin 1 and/or keratin 10 interaction partners, for example siRNAs against Runx1/Runx3. However, these *"indirect* inhibitors of protein expression of keratin 1 and/or keratin 10" may not be considered being comprised in the term "active agent" of the topical composition hereinunder but may be absent or only be used as additional excipients in the topical composition. Thus, in one embodiment the topical composition does not comprise indirect inhibitors of keratin 1 and/or keratin 10 protein expression, such as Runx1/Runx3 siRNAs, derivatives of vitamin A and/or methotrexate. In yet another embodiment the topical composition does only comprise indirect inhibitors of keratin 1 and/or keratin 10 protein expression, such as Runx1/Runx3 siRNAs, derivatives of vitamin A and/or methotrexate, as additional excipient additional to the active agent.
- Wherein the terms "suppresses keratin 1 (KRT1) and/or keratin 10 (KRT 10) in skin epidermis by inhibition of protein activity of keratin 1 and/or keratin 10" or "inhibition of the protein activity of keratin 1 and/or keratin 10" refers to an active substance which specifically reduces or completely prevents the protein activity of keratin 1 and/or keratin 10 in the target skin cell. The normal activity of keratin 1 and keratin 10 is to facilitate and maintain the structural integrity as structural proteins in epithelial cells. Thus, any interference of this activity is encompassed by said term. Examples for such specific inhibitors of protein activity may be proteins which bind to keratin 1 and/or keratin 10 thereby inhibiting the usual activity of said proteins. For example, specific inhibitors of protein activity may be antigen-binding compounds specifically binding to keratin 1 and/or keratin 10, which may be selected from the goup consisting of antibodies, T-cell rexeptors, B-cell receptors, fab-fragments, nanobodies, aptamers and/or peptide mimetics, and the like, as well as combinations thereof.
   Also, some compounds are reported which indirectly inhibit the protein activity of keratin 1 and/or keratin 10, e.g., by interfering with upstream proteins and/or keratin 1 and/or keratin 10 interaction partners, for example certain herbal extracts like green tea and licorice, Jagged1, and/or DAPT (N-[N-(3,5-Difluorphenacetyl)-L-alanyl]-S-phenylglycin-tert-butylester; a cell-permeable dipeptide that inhibits the protease γ-secretase inhibitor of Notch-1 signalling pathway). However, these *"indirect* inhibitors of protein activty of keratin 1 and/or keratin 10" may not be considered being comprised in the term "active agent" of the topical composition hereinunder but may be absent or only be used as additional excipients in the topical composition. Thus, in one embodiment the topical composition does not comprise indirect inhibitors of keratin 1 and/or keratin 10 protein activity, such as certain herbal extracts like green tea and licorice, Jagged1, and/or DAPT. In yet another embodiment the topical composition does only comprise indirect inhibitors of keratin 1 and/or keratin 10 protein activity, such as certain herbal extracts like green tea and licorice, Jagged1, and/or DAPT, as additional excipient additional to the active agent.
- Wherein the terms "suppresses keratin 1 (KRT1) and/or keratin 10 (KRT 10) in skin epidermis by degradation", "degradation of keratin 1 and/or keratin 10" or "degrades keratin 1 and/or keratin 10" refers herein to any break down of keratin 1 and keratin 10 by cleaving peptide bonds between amino acids. For example, proteases are involved in such a protein degradation.

Thus, in one embodiment the disclosure pertains to a topical composition for use in the treatment of seborrheic keratoses comprising an active agent which is selected from a group consisting of a siRNA specific for keratin 1 (KRT1) and/or keratin 10 (KRT 10), an antigen-binding compound specific for keratin 1 (KRT1) and/or keratin 10 (KRT 10), and a protease which degradates keratin 1 (KRT1) and/or keratin 10 (KRT 10), as well as any combination thereof.

The term "skin epidermis" is defined hereinunder as the outermost layer of the skin, composed of multiple layers of cells that provide a protective barrier against the external environment. The epidermis is made up of four main types of cells:
- Keratinocytes: These are the most abundant cells in the epidermis and produce the protein keratin, which gives the skin its strength and waterproofing properties.
- Melanocytes: These cells produce the pigment melanin, which helps to protect the skin from the damaging effects of the sun's ultraviolet radiation.
- Langerhans cells: These cells are involved in the immune response and help to protect the skin from infection.
- Merkel cells: These cells are involved in touch sensation and are located in the deepest layer of the epidermis.

Overall, the skin epidermis plays a critical role in maintaining the integrity of the skin and protecting the body from external stressors such as UV radiation, bacteria, and chemicals.

Seborrheic keratosis (SK) is a common benign skin growth. It is defined herein as a common, non-cancerous skin growth that typically appears as a raised, waxy, or scaly lesion. It is caused by the overgrowth of cells in the outer layer of the skin (epidermis) and is most commonly seen in older adults. SK can vary in colour from flesh-coloured to brown or black and can range in size from a few millimetres to several centimetres in diameter. Seborrheic keratosis may cause symptoms such as itching or irritation, or may be considered cosmetically bothersome.

One diagnostic test that may be used to identify seborrheic keratosis is a skin biopsy, in which a small sample of the growth is taken and examined under a microscope for characteristic features of seborrheic keratosis, such as the presence of keratin-filled cysts or horn cysts. Another test that may be used is dermoscopy, in which a specialized magnifying lens is used to examine the growth for specific features that suggest seborrheic keratosis, such as a "stuck-on" appearance, a "globular" or "milk-drop" appearance, or a "pseudo-horn cyst" or "pseudo-horn pearl" appearance, where there are multiple small, white, horn-like projections on the surface of the lesion.

Keratin 1 and keratin 10 are two types of proteins that are important structural components of the epidermis, which is the outermost layer of the skin. They belong to the family of intermediate filament proteins known as keratins, which provide mechanical support and protection to the skin. Keratins 1 and 10 are expressed in the keratinocytes of the epidermis, which are specialized cells that produce keratin and other structural proteins. Keratins 1 and 10 play a crucial role in forming the cytoskeleton of keratinocytes, which provides mechanical strength and stability to the skin.

In particular, keratins 1 and 10 are important in forming the "cornified envelope", which is a tough, protective layer that forms on the surface of the skin. This layer helps to prevent water loss, protects the skin from environmental stressors like UV radiation and chemicals, and provides a barrier against pathogens and other harmful substances.

Mutations in the genes that encode keratins 1 and 10 can lead to a variety of skin disorders, including ichthyosis, which is a group of inherited disorders characterized by thick, scaly skin. In addition, abnormalities in keratin 1 and 10 expression have been associated with various types of skin cancer, highlighting their importance in maintaining the integrity and function of the skin.

It has been surprisingly found that an inhibition and/or degradation of keratin 1 (KRT-1) and/or keratin 10 (KRT-10) allows the treatment of the seborrheic keratosis skin condition. Thus, the disclosed topical composition comprising at least one active agent which inhibits and/or degrades keratin 1 (KRT-1) and/or keratin 10 (KRT-10) allows the treatment of seborrheic keratosis.

As "active agent" any agent which inhibits the expression and/or inhibits the activity of and/or degrades KRT-1 and/or KRT-10 and is suitable to be formulated in a topical pharmaceutical composition. It can be a drug, a chemical compound, a biological product, or a natural substance. Active agents are the primary ingredient in pharmaceutical or therapeutically products and may be formulated in various ways as topical composition. The active agent is the component responsible for the intended therapeutically effect and is often accompanied by other components, such as excipients or additives, that help deliver or enhance its activity.

In one embodiment the active agent is selected from a group consisting of calpain; cathepsin L; a matrix metalloprotease (MMP), in one embodiment MMP-1 and/or MMP-9; a kallikrein, in one embodiment kallikrein-5 and/or kallikrein-7; a serine protease, in one embodiment elastase; a cysteine protease, in one embodiment papain; a lysosomal aspartic protease, in one embodiment cathepsin D; a phosphatase, in one embodiment ADP-sugar pyrophosphatase, and a caspase, in one embodiment caspase-14; and/or any combination thereof. In further embodiments the active agent may be selected from KRT1-siRNA, Jagged1, and DAPT (inhibitor of Notch-1 signalling pathway), or any other KRT-1 and/or KRT-10 inhibiting factor. In one preferred embodiment the active agent is selected from a group consisting of a serralysin family (metallo-) protease, in one embodiment from *Pseudomonas panacis;* and a hydrolase, in one embodiment from *Pseudomonas spec.;* and/or any combination thereof. In one embodiment the active agent is a serralysin family metalloprotease. In another embodiment the active agent is a hydrolase.

The indefinite article "a" or "an" as used hereinunder does not refer herein to the numeral "one", but also includes multiples. For example, "a serine protease", could be one serine protease, but also includes several serine proteases.

Further active agents which can be used in the disclosed topical composition include all agents, in one embodiment a protease, which achieve at least a score of 3 in the KRT1/KRT10-lysis test.

The KRT1/KRT10-lysis test may be performed with a direct ELISA KRT-1/KRT-10 test, wherein KRT-1 and KRT-10 is fixed to a surface (for example in a 96-well plate). 1 ug/ml of the active agent to be tested, for example a protease, is then dissolved in PBS-buffer at pH 7.4, at 20°C, and incubated for 1h on the surface with the fixated KRT1/KRT10. Then the surfaces are washed with wash buffer (0.05 % Tween 20 in PBS, pH 7.4). Then a normal KRT-1/10-ELISA-test is made, i.e., an anti-KRT-1/KRT-10 antibody coupled with an enzyme is applied (in PBS, pH 7.4, 20°C), incubated for 1.5h and unbound antibody is washed with wash buffer (0.05 % Tween 20 in PBS, pH 7.4, 20°C). The enzymatic colour reaction is started by adding a coloursubstrate for the enzyme (typical ELISA-test).

The absorbance at the respective wavelength is compared to a non-agent-treated reference under otherwise the same conditions (which represents the colour of 100% recognizable KRT-1/10).

The absorbance or reduction of absorbance, respectively, in comparison to the control then represents a score as follows:

| **Absorbance** | **Reduction of Absorbance in comparison to non-agent-treated reference** | **Score** |
|---|---|---|
| 100% (= same absorbance as non-agent-treated reference) | 0% | 0 |
| 90%-99% | 1-10% | 1 |
| 80%-89% | 11-20% | 2 |
| 70%-79% | 21-30% | 3 |
| 60%-69% | 31-40% | 4 |
| 50%-59% | 41-50% | 5 |
| 40%-49% | 51-60% | 6 |
| 30%-39% | 61-70% | 7 |
| 20%-29% | 71-80% | 8 |
| 10%-19% | 81-90% | 9 |
| 0%-9% | 91-100% | 10 |

Any score of 3 or better is considered a "positive test", e.g., a score of 4 or better, a score of 5 or better, a score of 6 or better, a score of 7 or better, a score of 8 or better, a score of 9 or better, or a score of 10, means that the active agent is suitable for use in the topical composition. Thus, in one embodiment the active agent is selected from an agent, in one embodiment a protease, which passed the KRT1/KRT10-lysis test with a score of at least 3. The advantage of the KRT1/KRT10-lysis test is that it represents an objectified in-vitro measurement, which is independent of natural fluctuations and may be used not only for selection of active ingredients, but also for quality control during production and pharmaceutical approval.

Further active agents which can be used in the disclosed topical composition include all agents, in one embodiment a protease, which achieve at least a score of 3 in in a lytic seborrheic keratosis cell cluster test.

The lytic seborrheic keratosis cell cluster test may be performed as follows: A single seborrheic keratosis is divided into halves of comparable surface, which are incubated for 1 week (168 hours, 20°C) in solutions containing or not containing the disclosed topical formation. Thereafter, seborrheic keratoses are placed between two glass slides and exposed to weight pressure of 1 kilogram for 24 hours (at temperature 20°C). Lysis is defined by comparing the percentage of the increase of area (in mm²) of the seborrheic keratosis exposed to the disclosed topical composition in comparison to the area (in mm²) of the seborrheic keratosis not exposed to the disclosed topical composition. The increase of area of the seborrheic keratosis exposed to the disclosed topical composition in comparison to the seborrheic keratosis not exposed to the disclosed topical composition then represents a score as follows:

| **Increase of area** | **Score** |
|---|---|
| more than 180% | 10 |
| 161%-180% | 9 |
| 141%-160% | 8 |
| 121%-140% | 7 |
| 101%-120% | 6 |
| 81%-100% | 5 |
| 61%-80% | 4 |
| 41-60% | 3 |
| 21%-40% | 2 |
| 11%-20% | 1 |
| 0%-10% (0% = same area as non-agent-treated reference) | 0 |

Any score of 3 or better is considered a "positive test", e.g., a score of 4 or better, a score of 5 or better, a score of 6 or better, a score of 7 or better, a score of 8 or better, a score of 9 or better, or a score of 10, means that the active agent is suitable for use in the topical composition. Thus, in one embodiment the active agent is selected from an agent, in one embodiment a protease, which passed the lytic seborrheic keratosis cell cluster test with a score of at least 3. One advantage of the lytic seborrheic keratosis cell cluster test is that the activity of the active agent can be tested on human tissue-material, in one embodiment on the tissue of the patient, which is to be treated, thereby avoiding and being able to adapt to natural fluctuations in patient-dependent efficiency. It may be used not only for selection of active ingredients, but also for dosage finding and individual dosage adaptation to patient's needs.

In one embodiment the active agent is selected from a group consisting of calpain; cathepsin L; a matrix metalloprotease (MMP), in one embodiment MMP-1 and/or MMP-9; a kallikrein, in one embodiment kallikrein-5 and/or kallikrein-7; a serine protease, in one embodiment elastase; a cysteine protease, in one embodiment papain; a lysosomal aspartic protease, in one embodiment cathepsin D; and a caspase, in one embodiment caspase-14; serralysin family metalloprotease and/or the hydrolase; and/or any combination thereof.

In yet a further embodiment the active agent the topical composition comprises at least a fraction of a microbial or fungal supernatant and/or lysate. It has been found that certain bacteria as well as fungi produce agents which inhibit and/or degrade KRT-1 and/or KRT-10. Thus, at least fractions of the microbial or fungal supernatant and/or lysate may be used as active agent in a topical composition according. In one embodiment, said fraction achieves a score of 3 in the KRT1/KRT10-lysis test disclosed herein. In yet a further embodiment the microbial and/or fungal supernatant and/or lysate is from a culture comprising an organism which is positive in a lytic seborrheic keratosis cell cluster test.

In yet a further embodiment the microbial or fungal supernatant and/or lysate is from a culture comprising an organism selected from a group consisting an organism of the family *pseudomonadaceae,* in one embodiment of the genera *pseudomonas* and/or *delftia;* an organism of the family *trichocomaceae,* in one embodiment of the genus *aspergillus;* an organism of the family *dipodascaceae,* in one embodiment of the genus *geotrichum;* an organism of the family *stereaceae,* in one embodiment of the genus *stereum,* and/or an organism of the family *trichocomaceae,* in one embodiment of the genus *paecilomyces,* in one embodiment of the species *P. marquandii;* and/or any combination thereof.

In one embodiment the selected bacterium and/or fungus is disclosed on the *"2023 list of QPS-recommended microorganisms for safety risk assessments carried out by EFSA"* (version 15; as of January 25, 2023; version-ID: 10.5281/zenodo.7554079).

In yet a further embodiment the topical composition is formulated as an ointment, a cream, a cream ointment, a gel, a hydrogel, a lotion, a solution, a fatty ointment, a powder, and/or a paste.

"Ointments" are defined herein as thick, greasy, and semisolid preparations that are mostly oilbased. They have high oil content and are designed to remain on the skin for a longer period. They are useful in treating dry and scaly skin conditions.

"Creams" are defined herein as lighter than ointments and are mostly water-based. They are easier to apply and absorb quickly into the skin. They are useful for treating mild skin conditions.

"Cream ointment" is defined herein is a combination of both ointment and cream. It has a higher oil content than cream but is lighter than an ointment. It is used for treating moderate to severe skin conditions.

"Gels" are defined herein as transparent, semisolid preparations that are mostly water-based. They are easily absorbed into the skin and are ideal for treating oily skin conditions.

"Hydrogels" are defined herein as similar to gels but have a higher water content. They are useful for treating wounds, and skin irritations.

"Lotions" are defined herein as lightweight and mostly water based. They are easy to apply and absorb quickly into the skin. They are useful for treating mild skin conditions.

"Solutions" are defined herein as liquid preparations that are mostly water- or ethanol-based. They penetrate or are quickly absorbed by the skin.

"Fatty ointment" is defined herein as being similar to ointments but have a higher fat content. They are useful for treating severe skin conditions.

"Powder" is defined herein as a dry preparation that can be applied to the skin to reduce friction and absorb moisture. It is useful for treating certain skin conditions.

"Paste" is defined herein as a thick and sticky preparation that is useful for treating skin conditions that require a barrier to protect the skin.

In summary, the main differences between these topical preparations are their consistency and the amount of oil or water they contain.

In one embodiment ointments typically have a high oil content, usually ranging from about 80 wt.-% to about 100 wt.-%, and a low water content, usually less than about 20 wt.-%. In further embodiments the oil content may be about 70 wt.-% or more, in one embodiment about 75 wt.-% or more, in one embodiment about 80 wt.-% or more and up to about 90 wt.-% or less, up to about 95 wt.-% or less, up to about 99 wt.-% or less, and up to about 100 wt.-%.

In another embodiment the creams are mostly water-based, with a ratio of oil to water of around about 50 wt.-% to about 50 wt.-%. In further embodiments the oil content may be about 45 wt.-% or more, in one embodiment about 48 wt.-% or more, in one embodiment about 49 wt.-% or more and up to about 52 wt.-% or less, up to about 54 wt.-% or less, up to about 55 wt.-% or less.

In another embodiment the cream ointments have a higher oil content as compared to creams, typically about 70 wt.-% oil and about 30 wt.-% water. In further embodiments the oil content may be about 60 wt.-% or more, in one embodiment about 65 wt.-% or more, and up to about 75 wt.-% or less, up to about 80 wt.-% or less.

In another embodiment the gels are mostly water-based, with a ratio of oil to water of around 5 wt.-% to about 95 wt.-%. In further embodiments the oil content may be about 1 wt.-% or more, in one embodiment about 2 wt.-% or more, in one embodiment about 3 wt.-% or more, in one embodiment about 4 wt.-% or more and up to about 6 wt.-% or less, up to about 7 wt.-% or less, up to about 8 wt.-% or less, and up to about 9 wt.-% or less, up to about 10 wt.-% or less.

In another embodiment the hydrogels have a high-water content, usually around 90 wt.-% or more, and a low oil content of less than 10 wt.-%. In further embodiments the oil content may be about 8 wt.-% or more, in one embodiment about 9 wt.-% or more, in one embodiment about 10 wt.-% or more, and up to about 12 wt.-% or less, up to about 13 wt.-% or less, up to about 14 wt.-% or less, and up to about 15 wt.-%.

In another embodiment the lotions are mostly water-based, with a ratio of oil to water of around 10 wt.-% to 90 wt.-%. In further embodiments the oil content may be about 8 wt.-% or more, in one embodiment about 9 wt.-% or more, in one embodiment about 10 wt.-% or more, and up to about 12 wt.-% or less, up to about 13 wt.-% or less, up to about 14 wt.-% or less, and up to about 15 wt.-%.

The difference between gel, lotion and hydrogel being that gels are usually thicker and more viscous than lotions, which are thinner and more liquid. Hydrogels are like gels but have a higher water content, which makes them more liquid and easier to spread. Gels are further typically water-based, and can contain other ingredients like alcohol, glycerine, or propylene glycol to help the active ingredients penetrate the skin. Lotions are also water-based but contain more oil than gels, which makes them more moisturizing. Hydrogels are also water-based but contain a higher percentage of water and less oil than gels, making them more lightweight and easier to absorb.

In another embodiment the solutions are mostly water-based, with a ratio of oil to water of around 10 wt.-% to 90 wt.-%. In further embodiments the oil content may be about 8 wt.-% or more, in one embodiment about 9 wt.-% or more, in one embodiment about 10 wt.-% or more, and up to about 12 wt.-% or less, up to about 13 wt.-% or less, up to about 14 wt.-% or less, and up to about 15 wt.-%.

In another embodiment the fatty ointments have a very high oil/fat content, usually around 90% to 100%, and a low water content. In further embodiments the oil content may be about 85 wt.-% or more, in one embodiment about 87 wt.-% or more, in one embodiment about 89 wt.-% or more, and up to about 95 wt.-% or less, up to about 98 wt.-% or less, up to about 99 wt.-% or less, and up to about 100 wt.-%. In one embodiment "fatty ointments" (also known as anhydrous ointment or oleaginous ointment) are free of water.

In another embodiment the powders are dry preparations and usually do not contain oil or water. However, the powders may comprise an absorbent, such as corn starch or talc; antifungal and antibacterial agents such as miconazole, clotrimazole, or zinc oxide; anti-inflammatory agents such as calamine or hydrocortisone; skin protectants, such as zinc oxide or petrolatum; exfoliants such as baking soda or kaolin clay.

In another embodiment the pastes have a high oil content, usually around 50% to 80%, and a low water content. In further embodiments the oil content may be about 35 wt.-% or more, in one embodiment about 40 wt.-% or more, in one embodiment about 45 wt.-% or more, and up to about 65 wt.-% or less, up to about 70 wt.-% or less, up to about 75 wt.-% or less, and up to about 80 wt.-%.

Please note that these ratios can vary depending on the specific composition and intended use of the topical preparation.

In one embodiment the topical composition is a sun cream or after sun lotion.

A typical topical composition may comprise in addition to the active agent:
- Ingredients such as Dimethyl sulfoxide (DMSO) to enhance the penetration and/or absorption of the topical preparation in seborrheic keratoses, making it more effective.
- Exfoliative ingredients such as salicylic acid, glycolic acid, lactic acid, and/or urea, as well as any combination thereof;
- Moisturizing ingredients such as glycerine, hyaluronic acid, aloe vera, and/or panthenol;
- Natural oils such as jojoba oil, almond oil, argan oil, and/or coconut oil;
- Vitamins such as vitamin E or vitamin C that can protect the skin from environmental damage;
- Agent to moisturize dry and cracked skin, such as for example shea butter;
- Agent to support cell renewal and improve the skin's appearance, such as for example lactic acid and/or fruit acids;
- Chamomile, allantoin, and/or bisabolol (INCI: BISABOLOL) to soothe and relax the skin;
- Collagen and/or elastin to improve skin structure and reduce the appearance of wrinkles;
- Antifungal and antibacterial agents such as miconazole, clotrimazole, triclosane, and/or zinc oxide;
- Anti-inflammatory agents such as calamine and/or hydrocortisone;
- Reducing agents like DTT for sulfitolysis (S-S bond breakage) in keratinolytic process;
- Skin protectants, such as zinc oxide and/or petrolatum;
- Exfoliants such as baking soda and/or kaolin clay; and/or
- Sunscreen ingredients such as zinc oxide and/or titanium dioxide to protect the skin from UV rays;
- As well as combinations thereof.

In a second aspect the present disclosure pertains also to a skin patch or a facemask containing the topical dosage form disclosed herein.

A "skin patch" is defined herein as a patch selected from the group consisting of topical patch, and/or hydrogel patch. In general, any patch which can be used for delivering medications, supplements, or other substances to the body is encompassed.

"Topical patches" are defined herein as a patch in order to deliver medication or supplements directly to the skin, rather than into the bloodstream. They are typically made up of a backing layer, an adhesive layer, and a layer containing the active ingredient. Topical patches can be used for a variety of purposes, such as pain relief, muscle relaxation, or delivering vitamins and minerals to the skin.

"Hydrogel patches" are defined herein as being made up of a hydrogel material that contains a drug or other active ingredient. They are designed to release the active ingredient slowly over time and are very beneficial for treating seborrheic keratosis.

The term "facemask" refers herein to a facemask for cosmetic and/or medical use, i.e., a skincare product that is applied to the face with the aim of applying the topical composition for a longer time onto the skin in the face-area. Face masks come in different forms, including sheet masks, clay masks, gel masks, and cream masks, and they are formulated with the active agent of the present invention as well as further excipients. Cosmetic face masks are typically designed to be used once or twice a week as part of a regular skincare routine. They are applied to the face and left on for a specified period before being rinsed off with warm water and/or taken off. In one embodiment the facemask may be made from bacterial nanocellulose (BNC).

In a third aspect the present disclosure pertains also to a method for producing the topical pharmaceutical composition, comprising the steps of
a. culturing an organism selected from a group consisting an organism of the family *pseudomonadaceae,* such as of the genera *pseudomonas* and/or *delftia;* an organism of the family *trichocomaceae,* such as of the genus *aspergillus;* an organism of the family *dipodascaceae,* such as of the genus *geotrichum;* an organism of the family *stereaceae,* such as of the genus *stereum,* and/or an organism of the family *trichocomaceae,* such as of the genus *paecilomyces,* such as of the species *P. marquandii* and/or an organism; and/or any combination thereof;
b. collecting the supernatant of the culture and/or the supernatant of a cell lysate of the culture;
c. optionally, concentrating and/or purifying the supernatant of step b.;
d. subjecting the supernatant of the culture and/or a supernatant of a cell lysate to a KRT1/KRT10-lysis test and/or lytic seborrheic keratosis cell cluster test and progressing only with a supernatant of the culture and/or a supernatant of a cell lysate which passed the KRT1/KRT10-lysis test and/or lytic seborrheic keratosis cell cluster test with a score of at least 3;
e. formulating the supernatant into a topical composition selected from the group consisting of an ointment, a cream, a cream ointment, a gel, a hydrogel, a lotion, a solution, a fatty ointment, a powder, and/or a paste;
f. optionally, adding further at least one compound selected from the group consisting of and further comprising at least one compound selected from the group consisting of DMSO, salicylic acid, glycolic acid, urea, glycerine, hyaluronic acid, aloe vera, panthenol, jojoba oil, almond oil, argan oil, coconut oil, vitamins such as vitamin E or vitamin C, shea butter, lactic acid, fruit acid, chamomile, allantoin, or bisabolol, collagen, elastin, antifungal and antibacterial agents such as miconazole, clotrimazole, triclosane, zinc oxide; anti-inflammatory agents such as calamine or hydrocortisone; DTT; skin protectants, such as zinc oxide or petrolatum; exfoliants such as baking soda or kaolin clay; and sunscreen ingredients such as zinc oxide or titanium dioxide, as well as any combination thereof;
g. thereby receiving a topical composition for the treatment of seborrheic keratosis.

There are protocols and commercially available kits, which the skilled person may use to grow the cells and collect the supernatant. However, in one embodiment the culturing conditions of step a. may comprise:
1. Culturing in nutrient agar or broth;
2. at temperatures between 25°C and 37°C;
3. at a pH between 6.5 and 7.5;
4. cultured in a well-ventilated environment;
5. with an incubation time typically around 24 to 48 hours.
6. Optionally selective media may be used, such as cetrimide agar or agar which specifically selects the required organism, such as for example *Pseudomonas* isolation agar.

The "collection" in step b. may be done by collecting the cells, washing and resuspending them with PBS, centrifugation of the sample at a high speed (typically, the sample is centrifuged at around 10,000 to 20,000 x g for 10 to 30 minutes); the pellet is at the bottom of the tube and the supernatant is the liquid portion above the pellet. The supernatant is carefully removed by using a pipette or other collection tool, taking care not to disturb the pellet. The supernatant is transferred to a new container and used for further processing. And/or, in case of a supernatant of a cell lysate of the culture, the cells are collected, washed with PBS (Phosphate Buffered Saline), resuspended in lysis buffer, then usually subjected to mechanical or chemical disruption to break open the cell membrane and release the contents of the cells. Methods of cell disruption may include sonication, grinding with a homogenizer, or repeated freeze-thaw cycles.

Thus, the term "cell lysate" as used herein refers to the mixture of cellular components obtained by breaking open or lysing cells. It typically contains a variety of macromolecules such as proteins, nucleic acids, lipids, and carbohydrates. The process of lysing cells to obtain the lysate can be achieved using various methods, including sonication, freeze-thaw cycles, and chemical lysis.

After the cells have been disrupted, the lysate is usually centrifuged to remove any unbroken cells, cell debris, and other solid components. The centrifugation typically separates the cell lysate into two components: the supernatant, which is the liquid portion containing the lysate, and the pellet, which is the solid portion containing any unbroken cells or debris. Finally, the supernatant is collected as described above.

The protein concentration and activity of the lysate can optionally be measured using various techniques such as Bradford assay, BCA assay, or enzymatic assays.

The "formulation step e." above may comprise the mixing of the supernatant directly with other excipients of the topical composition. In some embodiments additional treatment steps of the supernatant may be made, such as ultra-centrifugation in order to remove remaining debris; ultrafiltration in order to trap and remove smaller molecules; precipitation with ammonium sulfate or acetone in order to concentrate the protein; dialysis in order to remove smaller molecules and salts; and/or column chromatography in order to selectively separate and purify proteins from the supernatant. The skilled person will be able to select the method depending on the properties of the protein and the nature of the supernatant.

In a fourth aspect the present disclosure pertains kit comprising the topical pharmaceutical composition, and/or the skin patch and/or facemask, further comprising at least one other aid selected from an instruction leaflet, protection device and an applicator, as well as any combination thereof.

An "applicator" hereinunder is a tool or device used to apply the topical composition such as creams, gels, ointments, or lotions to the skin or other surfaces. The applicator may be selected from a tube; i.e., a plastic or metal tube commonly used for creams, gels, and ointments wherein the composition is squeezed out of the tube and applied directly to the skin or other surfaces; a syringe; ajar or pot: commonly used for thicker compositions such as creams or ointments wherein the composition is scooped out with a finger or spatula and applied to the skin; a spatula; a cotton swab; a roll-on-device which consists of a small rollerball that dispenses the composition onto the skin; a spray-device; a brush applicator which may be used for compositions that require precision application; a sponge; a pad and a dropper which is used for compositions that require precise dosage; as well as any combination thereof.

The "protection device" include any device suitable to protect the patient and/or the user (including any person applying the topic composition) from unwanted skin-contact with the topical composition, such as gloves, safety goggles, breathing mask, sterile pad or blanket, and the like.

In one embodiment, such kits may be primarily intended for medical laypersons, e.g., self-treatment by patients. However, in other embodiments, the kit is designed to be aimed at medical professionals such as doctors and nurses.

Thus, the "instruction leaflet" may comprise dosage regiments, application instructions, preparation instructions of the skin area to be treated, timing instructions, disposal instructions, and the like.

Thus, in one embodiment of the present disclosure the topical composition may comprise a therapeutically effective amount of an active agent which suppresses, i.e., inhibits the expression of and/or inhibits the activity of and/or degrades keratin 1 (KRT1) and/or keratin 10 (KRT 10) in skin epidermis for use in the treatment of a seborrheic keratosis.

In one embodiment the active agent may degrade keratin 1 (KRT1) and/or keratin 10 (KRT 10) in skin epidermis and may be selected from a group consisting of calpain; cathepsin L; a matrix metalloprotease (MMP), in one embodiment MMP-1 and/or MMP-9; a kallikrein, in one embodiment kallikrein-5 and/or kallikrein-7; a serine protease, in one embodiment elastase; a cysteine protease, in one embodiment papain; a lysosomal aspartic protease, in one embodiment cathepsin D; and a caspase, in one embodiment caspase-14; KRT1-siRNA, Jagged1; DAPT (inhibitor of Notch-1 signalling pathway); serralysin family protease, in one embodiment from *Pseudomonas panacis;* a hydrolase, in one embodiment from *Pseudomonas spec* or any other KRT-1 and/or KRT-10 inhibiting factor and/or any combination thereof. Wherein in a further embodiment the active agent may be selected from a compound, preferably a protease, which passed the KRT1/KRT10-lysis test and/or lytic seborrheic keratosis cell cluster test with a score of at least 3.

In one embodiment the active agent is and/or is derived from a supernatant of a culture of a microbial or fungal organism and/or a supernatant of a cell lysate of a culture of a microbial or fungal organism and wherein the active agent passed the KRT1/KRT10-lysis test and/or lytic seborrheic keratosis cell cluster test with a score of at least 3. Thus, in some embodiments, the "active agent" may be a supernatant and as such a mixture of one or more different active agents. Thus, the supernatant may be used which passed the KRT1/KRT10-lysis test and/or lytic seborrheic keratosis cell cluster test with a score of at least 3. Optionally the supernatant may be purfied and/or concentrated before being subjected to the KRT1/KRT10-lysis test and/or lytic seborrheic keratosis cell cluster test in order to improve the score to at least 3.

In one embodiment the topical composition comprises a supernatant of a *delftia* (genus of Gram-negative bacteria) cell culture, in one embodiment comprising a serralysin family metalloprotease. In another embodiment the topical composition comprises a supernatant of a *geotrichum* (a genus of fungi) cell culture, in one embodiment comprising a serralysin family metalloprotease. In yet another embodiment the topical composition further comprises papain and/or another metalloprotease such as MMP1 or MMP9. In yet another embodiment the topical formulation may comprise a least one additive selected from at least 10%, at least 20%, at least 30%, at least 40%, at least 50% (vol/vol) DMSO, at least 0.1 M, at least 0.25 M, at least 0.5 M, at least 0.75 M, at least 1 M DTT, and at least 0.5%, at least 1%, at least 2%, at least 5%, at least 10%, at least 20%, at least 30%, at least 40% (wt/vol) salicylic acid, as well as any combination thereof. In yet another embodiment the topical formulation may comprise a least one additive selected from up to 20%, up to 30%, up to 40%, up to 50%, up to 60%, up to 70%, up to 80%, (vol/vol) DMSO, up to 0.3 M, up to 0.6 M, up to 0.8 M, up to 1 M DTT, and up to 0.75%, up to 3%, up to 5%, up to 10%, up to 20%, up to 40%, up to 60%, up to 80% (wt/vol) salicylic acid, as well as any combination thereof. In yet another embodiment the topical formulation may comprise a least one additive selected between about 10% - 80%, between about 20% - 70%, between about 30% - 60%, between about 40% - 50% (vol/vol) DMSO, between about 0.1 - 1 M, between about 0.3 - 0.8 M, between about 0.4 - 0.75 M DTT, and between about 0.75% - 80%, between about 2% - 70%, between about 5% - 50%, between about 10% - 40%, between about 20% - 30% (wt/vol) salicylic acid, as well as any combination thereof. In another embodiment the topical composition may be a fatty ointment. In another embodiment the topical composition may be comprised in a skin patch.

Thus, in exemplary embodiment of the topical composition may be selected from:
- Fatty ointment comprising a protease selected from a serralysin family metalloprotease, papain, and a metalloprotease such as MMP1 or MMP9, as well as any combination thereof;
- Fatty ointment comprising a protease selected from a serralysin family metalloprotease, papain, and a metalloprotease such as MMP1 or MMP9, as well as any combination thereof; further comprising an additive selected from DMSO, DTT, and salicylic acid, as well as any combination thereof;
- Fatty ointment comprising a protease selected from a serralysin family metalloprotease, papain, and a metalloprotease such as MMP1 or MMP9, as well as any combination thereof; further comprising an additive selected from at least 10%, at least 20%, at least 30%, at least 40%, at least 50% (vol/vol) DMSO, at least 0.1, at least 0.25, at least 0.5, at least 0.75, at least 1 M DTT, and at least 0.5%, at least 1%, at least 2%, at least 5%, at least 10%, at least 20%, at least 30%, at least 40% (wt/vol) salicylic acid, as well as any combination thereof;
- Fatty ointment comprising a supernatant of a *delftia* culture and/or cell lysate;
- Fatty ointment comprising a supernatant of a *delftia* culture and/or cell lysate, comprising protease selected from a serralysin family metalloprotease, papain, and a metalloprotease such as MMP1 or MMP9, as well as any combination thereof;
- Fatty ointment comprising a supernatant of a *delftia* culture and/or cell lysate, comprising an additive selected from DMSO, DTT, and salicylic acid, as well as any combination thereof;
- Fatty ointment comprising a supernatant of a *delftia* culture and/or cell lysate, comprising a protease selected from a serralysin family metalloprotease, papain, and a metalloprotease such as MMP1 or MMP9, as well as any combination thereof; further comprising an additive selected from DMSO, DTT, and salicylic acid, as well as any combination thereof;
- Fatty ointment comprising a supernatant of a *delftia* culture and/or cell lysate, comprising a protease selected from a serralysin family metalloprotease, papain, and a metalloprotease such as MMP1 or MMP9, as well as any combination thereof; further comprising an additive selected from at least 10%, at least 20%, at least 30%, at least 40%, at least 50% (vol/vol) DMSO, at least 0.1, at least 0.25, at least 0.5, at least 0.75, at least 1 M DTT, and at least 0.5%, at least 1%, at least 2%, at least 5%, at least 10%, at least 20%, at least 30%, at least 40% (wt/vol) salicylic acid, as well as any combination thereof;
- Fatty ointment comprising a supernatant of a *geotrichum* culture and/or cell lysate;
- Fatty ointment comprising a supernatant of a *geotrichum* culture and/or cell lysate, comprising protease selected from a serralysin family metalloprotease, papain, and a metalloprotease such as MMP1 or MMP9, as well as any combination thereof;
- Fatty ointment comprising a supernatant of a *geotrichum* culture and/or cell lysate, comprising an additive selected from DMSO, DTT, and salicylic acid, as well as any combination thereof;
- Fatty ointment comprising a supernatant of a *geotrichum* culture and/or cell lysate, comprising protease selected from a serralysin family metalloprotease, papain and metalloprotease such as MMP1 or MMP9, as well as any combination thereof; further comprising an additive selected from DMSO, DTT and salicylic acid, as well as any combination thereof;
- Fatty ointment comprising a supernatant of a *geotrichum* culture and/or cell lysate, comprising a protease selected from a serralysin family metalloprotease, papain, and a metalloprotease such as MMP1 or MMP9, as well as any combination thereof; further comprising an additive selected from at least 10%, at least 20%, at least 30%, at least 40%, at least 50% (vol/vol) DMSO, at least 0.1, at least 0.25, at least 0.5, at least 0.75, at least 1 M DTT, and at least 0.5%, at least 1%, at least 2%, at least 5%, at least 10%, at least 20%, at least 30%, at least 40% (wt/vol) salicylic acid, as well as any combination thereof;
- Skin patch comprising a protease selected from a serralysin family metalloprotease, papain and metalloprotease such as MMP1 or MMP9, as well as any combination thereof;
- Skin patch comprising a protease selected from a serralysin family metalloprotease, papain and metalloprotease such as MMP1 or MMP9, as well as any combination thereof; further comprising an additive selected from DMSO, DTT, and salicylic acid, as well as any combination thereof;
- Skin patch comprising a protease selected from a serralysin family metalloprotease, papain, and a metalloprotease such as MMP1 or MMP9, as well as any combination thereof; further comprising an additive selected from at least 10%, at least 20%, at least 30%, at least 40%, at least 50% (vol/vol) DMSO, at least 0.1, at least 0.25, at least 0.5, at least 0.75, at least 1 M DTT, and at least 0.5%, at least 1%, at least 2%, at least 5%, at least 10%, at least 20%, at least 30%, at least 40% (wt/vol) salicylic acid, as well as any combination thereof;
- Skin patch comprising a supernatant of a *delftia* culture and/or cell lysate;
- Skin patch comprising a supernatant of a *delftia* culture and/or cell lysate, comprising a protease selected from a serralysin family metalloprotease, papain, and a metalloprotease such as MMP1 or MMP9, as well as any combination thereof;
- Skin patch comprising a supernatant of a *delftia* culture and/or cell lysate, comprising an additive selected from DMSO, DTT, and salicylic acid, as well as any combination thereof;
- Skin patch comprising a supernatant of a *delftia* culture and/or cell lysate, comprising a protease selected from a serralysin family metalloprotease, papain, and a metalloprotease such as MMP1 or MMP9, as well as any combination thereof; further comprising an additive selected from DMSO, DTT, and salicylic acid, as well as any combination thereof;
- Skin patch comprising a supernatant of a *delftia* culture and/or cell lysate, comprising a protease selected from a serralysin family metalloprotease, papain, and a metalloprotease such as MMP1 or MMP9, as well as any combination thereof; further comprising an additive selected from at least 10%, at least 20%, at least 30%, at least 40%, at least 50% (vol/vol) DMSO, at least 0.1, at least 0.25, at least 0.5, at least 0.75, at least 1 M DTT, and at least 0.5%, at least 1%, at least 2%, at least 5%, at least 10%, at least 20%, at least 30%, at least 40% (wt/vol) salicylic acid, as well as any combination thereof;
- Skin patch comprising a supernatant of a *geotrichum* culture and/or cell lysate;
- Skin patch comprising a supernatant of a *geotrichum* culture and/or cell lysate, comprising a protease selected from a serralysin family metalloprotease, papain, and a metalloprotease such as MMP1 or MMP9, as well as any combination thereof;
- Skin patch comprising a supernatant of a *geotrichum* culture and/or cell lysate, comprising an additive selected from DMSO, DTT, and salicylic acid, as well as any combination thereof;
- Skin patch comprising a supernatant of a *geotrichum* culture and/or cell lysate, comprising a protease selected from a serralysin family metalloprotease, papain, and a metalloprotease such as MMP1 or MMP9, as well as any combination thereof; further comprising an additive selected from DMSO, DTT, and salicylic acid, as well as any combination thereof;
- Skin patch comprising a supernatant of a *geotrichum* culture and/or cell lysate, comprising a protease selected from a serralysin family metalloprotease, papain, and a metalloprotease such as MMP1 or MMP9, as well as any combination thereof; further comprising an additive selected from at least 10%, at least 20%, at least 30%, at least 40%, at least 50% (vol/vol) DMSO, at least 0.1, at least 0.25, at least 0.5, at least 0.75, at least 1 M DTT, and at least 0.5%, at least 1%, at least 2%, at least 5%, at least 10%, at least 20%, at least 30%, at least 40% (wt/vol) salicylic acid, as well as any combination thereof;

In yet another embodiment the disclosure pertains to the use of the topical composition, wherein the topical composition may comprise a supernatant of a culture of a microbial or fungal organism and/or a supernatant of a cell lysate of a culture of a microbial or fungal organism, comprising an active agent which degradates keratin 1 (KRT1) and/or keratin 10 (KRT 10). In one embodiment the organism may be selected from a group consisting an organism of the family *pseudomonadaceae,* such as of the genera *pseudomonas* and/or *delftia;* an organism of the family *trichocomaceae,* such as of the genus *aspergillus;* an organism of the family *dipodascaceae,* such as of the genus *geotrichum;* an organism of the family *stereaceae,* such as of the genus *stereum,* and/or an organism of the family *trichocomaceae,* such as of the genus *paecilomyces,* such as of the species *P. marquandii;* and/or any combination thereof.

In yet another embodiment the topical composition may comprise a therapeutically effective amount of an active agent which degrades keratin 1 (KRT1) and/or keratin 10 (KRT 10) in skin epidermis, wherein in another embodiment the active agent may be selected from a group consisting of calpain; cathepsin L; a matrix metalloprotease (MMP), in one embodiment MMP-1 and/or MMP-9; a kallikrein, in one embodiment kallikrein-5 and/or kallikrein-7; a serine protease, in one embodiment elastase; a cysteine protease, in one embodiment papain; a lysosomal aspartic protease, in one embodiment cathepsin D; and a caspase, in one embodiment caspase-14; KRT1-siRNA, Jagged1 ; DAPT (inhibitor of Notch-1 signalling pathway); serralysin family protease, in one embodiment from *Pseudomonas panacis;* a hydrolase, in one embodiment from *Pseudomonas spec* or any other KRT-1 and/or KRT-10 inhibiting factor and/or any combination thereof, wherein in a further embodiment the active agent comprises at least fractions of a microbial or fungal supernatant and/or lysate.

In one further embodiment the organism which supernatant may used for the topical composition may be disclosed in the "2023 list of QPS-recommended micro-organisms for safety risk assessments carried out by EFSA" (version 15; as of January 25, 2023; version-ID: 10.5281/zenodo.7554079). The organisms disclosed in that list do have the advantage of being harmless when ingested or brought in contact with the human body.

In one embodiment the microbial or fungal supernatant and/or cell lysate may be from a culture comprising an organism which is positive in a KRT1 /KRT10-lysis test and/or a lytic seborrheic keratosis cell cluster test, in one embodiment the supernatan shows at least a score of 3 in a KRT1 /KRT10-lysis test and/or a lytic seborrheic keratosis cell cluster test.

In one further embodiment the topical composition may be formulated as an ointment, a cream, a cream ointment, a gel, a hydrogel, a lotion, a solution, a fatty ointment, a powder, and/or a paste; and in a further embodiment may comprise at least one compound selected from the group consisting of DMSO, salicylic acid, glycolic acid, urea, glycerin, hyaluronic acid, aloe vera, panthenol, jojoba oil, almond oil, argan oil, coconut oil, vitamins such as vitamin E or vitamin C, shea butter, lactic acid, fruit acid, chamomile, allantoin, or bisabolol, collagen, elastin, antifungal and antibacterial agents such as miconazole, clotrimazole, triclosane, zinc oxide; anti-inflammatory agents such as calamine or hydrocortisone; DTT; skin protectants, such as zinc oxide or petrolatum; exfoliants such as baking soda or kaolin clay; and sunscreen ingredients such as zinc oxide or titanium dioxide, as well as any combination thereof.

In another further embodiment a skin patch and/or facemask containing the topical dosage form according is disclosed.

In yet another embodiment a method for producing the topical composition is disclosed, comprising at least the steps of:
a. culturing an organism selected from a group consisting an organism of the family *pseudomonadaceae,* such as of the genera *pseudomonas* and/or *delftia;* an organism of the family *trichocomaceae,* such as of the genus *aspergillus;* an organism of the family *dipodascaceae,* such as of the genus *geotrichum;* an organism of the family *stereaceae,* such as of the genus *stereum,* and/or an organism of the family *trichocomaceae,* such as of the genus *paecilomyces,* such as of the species *P. marquandii* and/or an organism; and/or any combination thereof;
b. collecting a supernatant of the culture and/or a supernatant of a cell lysate of the culture;
c. optionally, concentrating and/or purifying the supernatant of step b.;
d. subjecting the supernatant of the culture and/or a supernatant of a cell lysate to a KRT1/KRT10-lysis test and/or lytic seborrheic keratosis cell cluster test and progressing only with a supernatant of the culture and/or a supernatant of a cell lysate which passed the KRT1/KRT10-lysis test and/or lytic seborrheic keratosis cell cluster test with a score of at least 3;
e. formulating the supernatant into a topical composition selected from the group consisting of an ointment, a cream, a cream ointment, a gel, a hydrogel, a lotion, a solution, a fatty ointment, a powder, and/or a paste;
f. optionally, adding further at least one compound selected from the group consisting of DMSO, salicylic acid, glycolic acid, urea, glycerin, hyaluronic acid, aloe vera, panthenol, jojoba oil, almond oil, argan oil, coconut oil, vitamins such as vitamin E or vitamin C, shea butter, lactic acid, fruit acid, chamomile, allantoin, or bisabolol, collagen, elastin, antifungal and antibacterial agents such as miconazole, clotrimazole, triclosane, zinc oxide; anti-inflammatory agents such as calamine or hydrocortisone; DTT; skin protectants, such as zinc oxide or petrolatum; exfoliants such as baking soda or kaolin clay; and sunscreen ingredients such as zinc oxide or titanium dioxide, as well as any combination thereof;
g. thereby receiving a topical composition for the treatment of seborrheic keratosis.

In yet another embodiment a kit for the use disclosed herein, comprising the topical pharmaceutical composition disclosed herein, and/or the skin patch and/or facemask disclosed herein, and/or further comprising at least one other aid selected from an instruction leaftlet, a protection device and an applicator; as well as any combination thereof.

The embodiments of the present application are further exemplified by the examples outlined in the example section.

### Description of the Figures

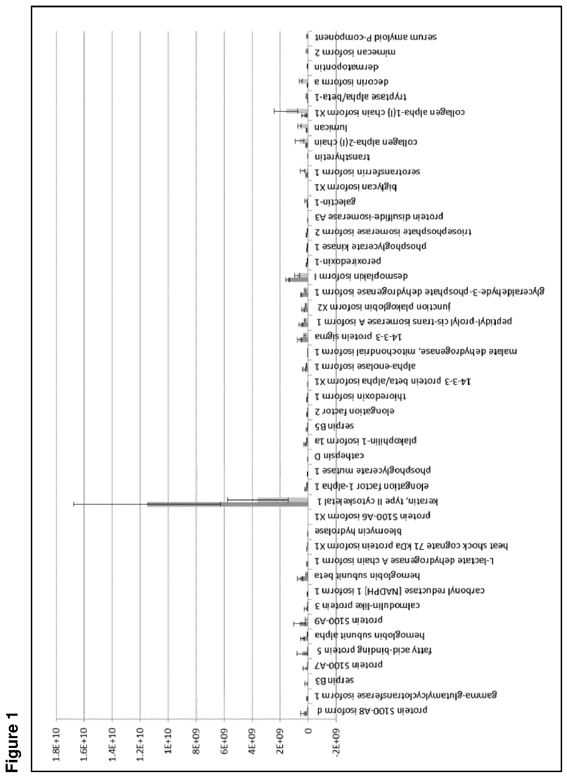

**FIGURE 1** - **Proteomics reveals highly expressed proteins in seborrheic keratoses.** Mass spectrometry analyzed proteins of four seborrheic keratoses (dark bars on the left) in comparison to four healthy skin specimens (bright bars on the right). LFQ (label free quantitation) intensities revealed 46 proteins statistically differentially regulated, listed logarithmic in this bar diagram with mean values und standard deviations. Among them, keratin 1 (keratin, type II cytoskeletal 1) accounts for the leading portion of regulated protein. Mass spectrometry was performed by Proteome Factory AG, Berlin, using an Ultimate 3000 nanoHPLC system as described previously (Schiller-HB et al. Am J Resp and Crit Care Med 2017, 196(10):1298; Simabuco-FM et. al. Mol Cancer 2014, 13:24). Data were analyzed with MaxQuant/Perseus. Quantitation by emPAI protocol (Ishihama-Y et al. Mol. and Cell. Proteomics 2005, 4:1272) reveals keratin 1 (keratin, type II, cytoskeletal 1) higher expressed in the four seborrheic keratoses compared to the four corresponding healthy skin specimens (LFQ 14.4 vs. 5.8; 28.1 vs. 8.7; 30.9 vs. 3.7; 9.7 vs. 2.6), which was statistically significant. Keratin 10 (keratin, type I, cytoskeletal 10) was even higher expressed in the four seborrheic keratoses compared to the four corresponding healthy skin specimens (LFQ 31 vs. 8.4; 67.7 vs. 20.3; 61 vs. 3.8; 11.1 vs. 2.9), which was not statistically significant and therefore is not included in this bar diagram.

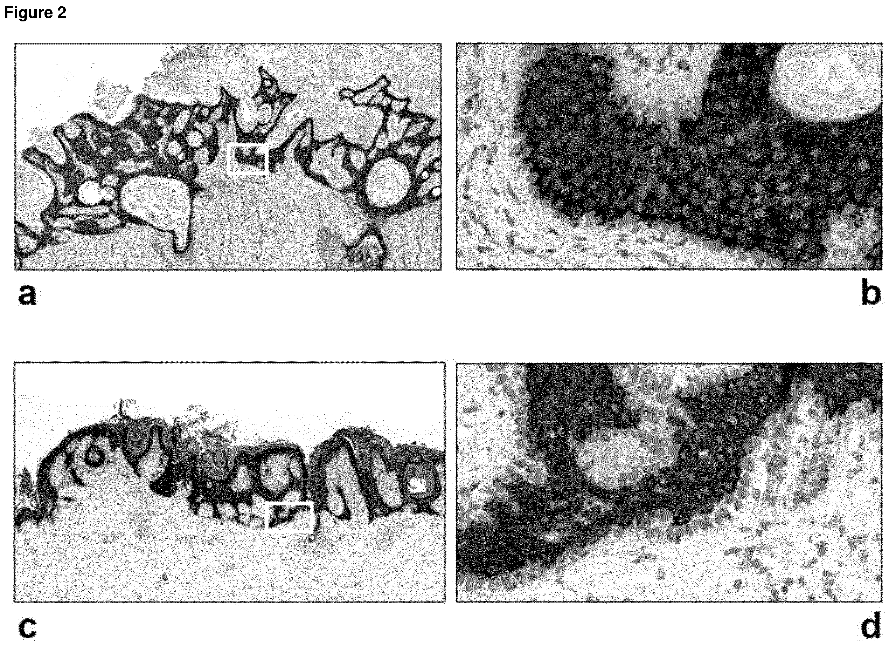

**FIGURE 2** - **Keratin 1 and 10 immunohistochemical staining of seborrheic keratoses.** Seborrheic keratoses are throughout positive for keratin 1 (a, antibody LHK1, NB100-2756, Novusbio) and largely positive for keratin 10 (c, antibody M7002, Clone DE-K10, Agilent), appearing as dark staining of cell cytoplasma. Immunhistochemistry was negative for dermis (a, c), except adnexa, and was negative for basal cell layer of epithelia (b as enlarged segment of a, d as enlarged segment of c), so that lysis of seborrheic keratoses by targeting keratin 1 and/or keratin 10 is not expected either to damage dermis nor to induce scars. A total of 8 seborrheic keratoses were investigated by histochemistry standard procedures (Wiedemeyer-K et al. Am J Surg Pathol 2020, 44(5):711; Ferreira-I et al. Histopathology 2018, 72(4):679).

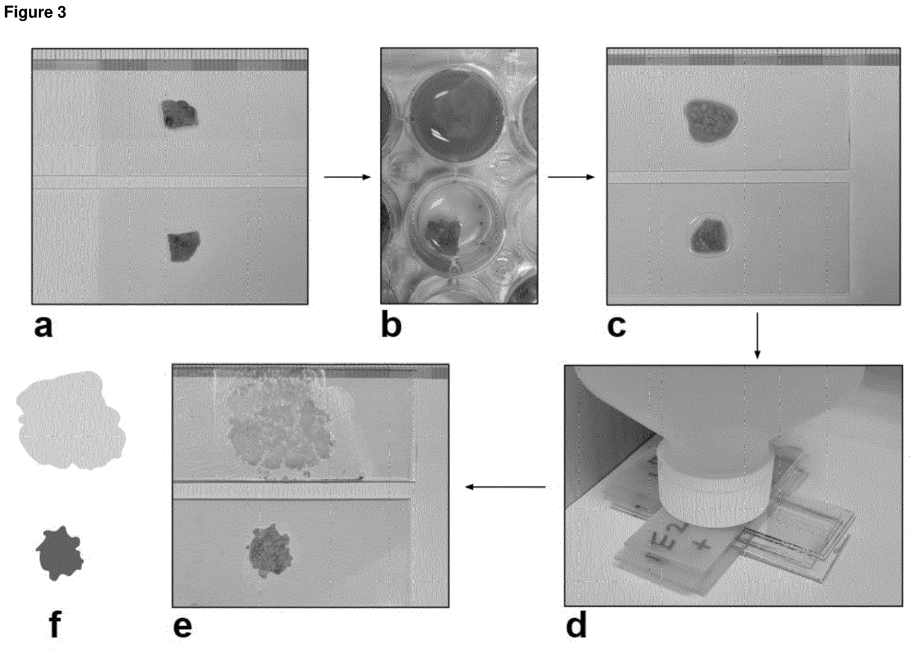

**FIGURE 3** - **Lytic seborrheic keratosis cell cluster test with microbiologic supernatants.** Seborrheic keratosis divided in two halves (a) were incubated in 24 well plates with Sabouraud broth liquid bouillon without antibiotics for 168 hours by room temperature (b), the upper half cultured with *delftia* (specified by 16S rDNA sequencing, DSMZ, Braunschweig, Germany). Pressed between two glasses by 1 kilogram for 24 hours (d), area of upper half incubated with *delftia* increased 530% in comparison to lower half incubated in bouillon without *delftia* (e, f), resulting in lytic seborrheic keratosis cell cluster test score 10. Lytic seborrheic keratosis cell cluster test with three further seborrheic keratoses resulted in scores 10, 10, and 9.

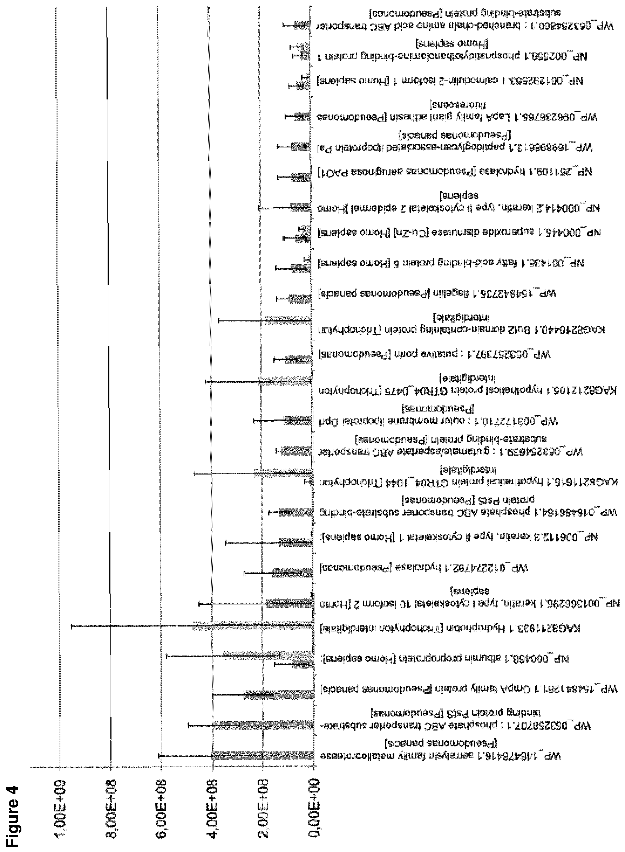

**FIGURE 4** - **Supernatants of successful lytic seborrheic keratosis cell cluster tests analyzed by mass spectrometry.** Supernatants of seborrheic keratoses lysed successfully in experiment described in figure 3 (dark bars on the left) were then compared to failed tests (bright bars on the right) by mass spectrometry as described in figure 1. 25 proteins with highest LFQ (label free quantitation) differences are specified among them keratin 1 (keratin, type II, cytoskeletal 1) and keratin 10 (keratin, type I, cytoskeletal 10). Retrieving these seborrheic keratoses target proteins in the supernatants, proteomics confirms successful breakage of seborrheic keratoses. Among the proteins with highest LFQ intensities, serralysin family metallprotease (bar in leftmost position) reveals as possible protease candidate for seborrheic keratoses.

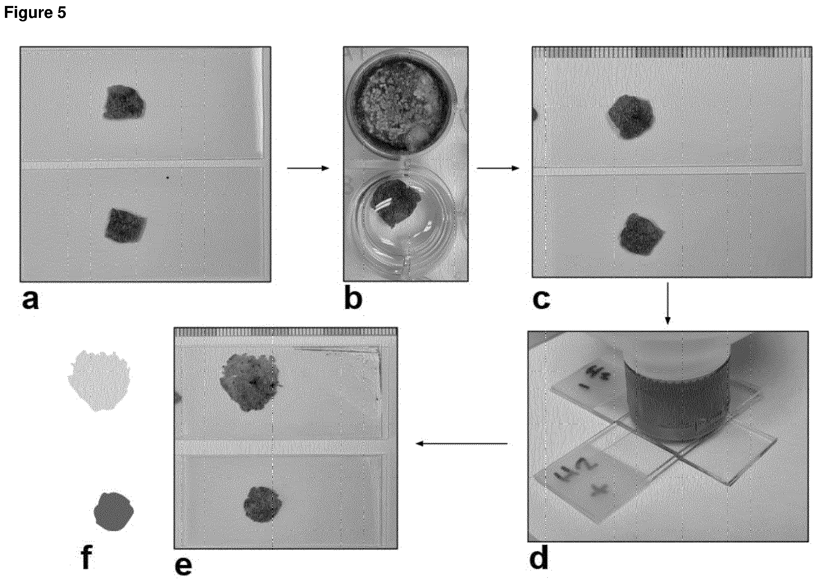

**FIGURE 5** - **Lytic seborrheic keratosis cell cluster test with fungal supernatants.** Seborrheic keratosis divided in two halves (a) were incubated in 24 well plates with Sabouraud broth liquid bouillon supplemented with antibiotics gentamycin 50ug/ml and chloramphenicol 500ug/ml for 144 hours by room temperature (b), the upper half cultured with geotrichum (specified by 18s ITS sequencing, NRZMyk, Würzburg). Pressed between two glasses by 1 kilogram for 16 hours (d), area of upper half incubated with *geotrichum* increased 201% in comparison to lower half incubated in bouillon without *geotrichum* (e, f), resulting in lytic seborrheic keratosis cell cluster test score 10. Further experiments with *paecilomyces, trichocomaceae, dipodascaceae* and *stereaceae* resulted in scores 6, 10, 5, and 7.

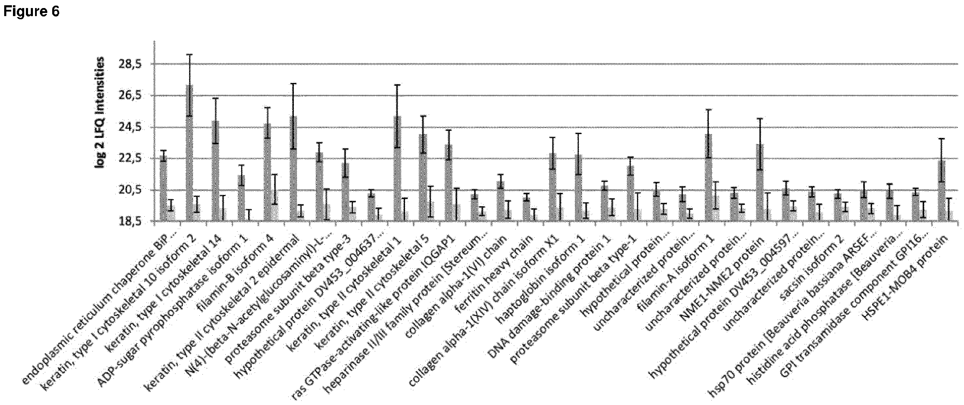

**FIGURE 6** - **Supernatants of successful lytic seborrheic keratosis cell cluster tests analyzed by mass spectrometry.** Supernatants of seborrheic keratoses lysed successfully in experiment described in figure 5 (dark bars on the left) were then compared to failed tests (bright bars on the right) by proteomics as described in figure 1, involving gauss imputation (to avoid incomplete statistical analysis resulting from zero point LFQ values), exhibiting 80 proteins significantly augmented protein intensities in LFQ (label free quantification), the first 31 beeing specified in this bar diagram, among them keratin 1 (keratin, type II cytoskeletal 1) and keratin 10 (keratin, type I, cytoskeletal 10), the target proteins to lyse seborrheic keratoses. In conclusion, proteomics of supernatants confirms breakage of seborrheic keratoses.

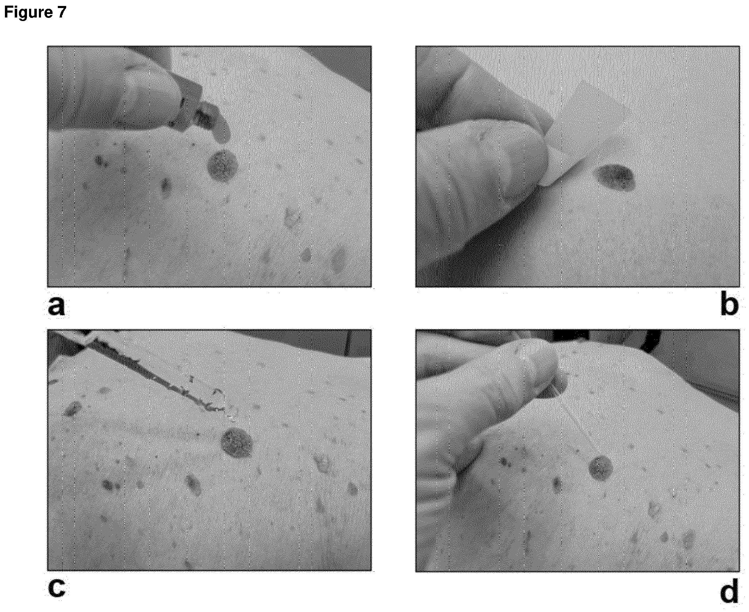

**FIGURE 7** - **Exemplary treatment of seborrheic keratoses on skin.** Seborrheic keratoses are treated with topical pharmaceutical compositions of ointment (a), solution (b, c), and patch (d), containing a therapeutically effective amount of an active agent which degrades keratin 1 (KRT 1) and/or keratin 10 (KRT 10), with the purpose of removal.

### Examples

### Example 1. Mass spectroscopy experimental setup and results of seborrheic keratosis skin and healthy skin with respect to KRT 1 and 10.

Four seborrheic keratoses and four corresponding healthy skin specimens were conducted to protein extraction. Each tissue sample was homogenized with liquid nitrogen using mortar and pestle. Tissue protein from each sample was resuspended in six volume of preparation buffer (9 M urea, 70mM DTT) and incubated at room temperature for 30 min under agitation. After centrifugation at 10,000g for 30 min at 4°C, the supernatant was quantified using Bradford assay. Protein solutions were then analyzed by mass spectrometry, using an Ultimate 3000 nanoHPLC system as described previously (Schiller-HB et al. Am J Resp and Crit Care Med 2017, 196(10):1298; Simabuco-FM et. al. Mol Cancer 2014, 13:24). Shortly, liquots of 400 µg proteolytic peptides were loaded onto a trapping column (kept at 35°C) for desalting (Thermo, Dionex, Pepmap C18), then peptides were separated by gradient elution on a 500x0.075mm column (0.5 µl/min, kept at 50°C, Reprosil C18-AQ, Dr. Maisch) from 8% to 40% acetonitrile supplemented with 0.1% formic acid. The column effluent was directed to an Orbitrap Velos mass spectrometer via a nano electrospray ion source. Survey scans were detected at a nominal resolution of R=60,000, and up to ten MS/MS spectra from ions of interest (charge states +2 and above) were data-dependently recorded in the instrument's linear ion trap. Total acquisition time was 150 min per analysis. Data were analyzed with MaxQuant/Perseus. Database search was done against human sequences. Default settings were used for further parameters. Protein identification results were loaded into the Perseus software (version 1.6.14.0). Two protein groups were used for statistical analysis. After grouping und log2 transformation of the LFQ (label free quantitation) intensities, protein groups were filtered to contain at least five (three) valid values in at least one group. Furthermore, the analysis was carried out once with and once without imputed missing values. Missing values in respective samples were imputed from a normal distribution with a width of 0.3 and a downshift of 1.5 based on the assumption of missing values being a result of the detection limit of the mass spectrometer used and not missing randomly. For the statistical comparison of the groups s0 = 0.1, FDR 0.5 was used. In results, LFQ intensities revealed 46 proteins statistically differentially regulated. Among them, keratin 1 (keratin, type II cytoskeletal 1) accounted for the leading portion of regulated protein. Quantitation by emPAI protocol (Ishihama-Y et al. Mol. and Cell. Proteomics 2005, 4:1272) reveals keratin 1 (keratin, type II, cytoskeletal 1) higher expressed in the four seborrheic keratoses compared to the four corresponding healthy skin specimens (LFQ 14.4 vs. 5.8; 28.1 vs. 8.7; 30.9 vs. 3.7; 9.7 vs. 2.6), which was statistically significant. Keratin 10 (keratin, type I, cytoskeletal 10) was even higher expressed in the four seborrheic keratoses compared to the four corresponding healthy skin specimens (LFQ 31 vs. 8.4; 67.7 vs. 20.3; 61 vs. 3.8; 11.1 vs. 2.9), which was not statistically significant.

### Example 2. Immunohistological experimental setup and results

A total of 9 seborrheic keratoses were investigated by histochemistry standard procedures. All tissue had been fixed in formalin, embedded in paraffin, and immunohistochemical stains were performed with the alkaline phosphatase/anti-alkaline phosphatase (APAAP) method according to standard procedures previously described (Wiedemeyer-K et al. Am J Surg Pathol 2020, 44(5):711; Ferreira-I et al. Histopathology 2018, 72(4):679; Stein-P et al. J Dermatol Sci 2015, 78(1):26). Antibodies against the antigens keratin 1 (antibody LHK1, NB100-2756, Novusbio) and keratin 10 (antibody M7002, Clone DE-K10, Agilent) were used according to the manufacturers' protocols. Shortly, sections were dewaxed, re-hydrated, and then incubated with primary antibody diluted in phosphate-buffered saline (PBS) at pH 7.4 for 20 minutes, secondary rabbitanti-mouse IgG for 30 minutes, APAAP kit (mouse) (Dako) for 30 minutes, and finally with substrate solution alkaline phosphatase containing naphthol AS-MX phosphate/Fast Red TR as the chromogen. Mayer's haematoxylin was used as the counterstain and the sections were mounted in an aqueous medium (Glycergel; Dako). In results, seborrheic keratoses were throughout positive for keratin 1 and largely positive for keratin 10, appearing as red staining of cell cytoplasm. Immunohistochemistry was negative for dermis, except adnexa, and was negative for basal cell layer of epithelia, so that lysis of seborrheic keratoses by targeting keratin 1 and/or keratin 10 is not expected either to damage dermis nor to induce scars.

### Example 3. Delftia-culture supernatant tests, experimental setup and results

Four seborrheic keratoses were divided in two halves and incubated in 24 well plates with Sabouraud broth liquid bouillon without antibiotics for 168 hours by room temperature, one half cultured with *delftia* (specified by 16S rDNA sequencing, DSMZ, Braunschweig, Germany), the other half cultured without *delftia.* A fifth seborrheic keratosis was divided in two halves and incubated in 24 well plates with Sabouraud broth liquid bouillon without antibiotics for 168 hours by room temperature, one half cultured with trichophyton, the other half cultured without trichophyton. A sixth seborrheic keratosis was divided in two halves and incubated in 24 well plates with Sabouraud broth liquid bouillon without antibiotics for 168 hours by room temperature, one half cultured with klebsiella, the other half cultured without klebsiella.

Thereafter pressed between two glasses by 1 kilogram for 24 hours, area of halves incubated with *delftia* increased significantly in comparison to halves incubated in bouillon without *delftia,* resulting in lytic seborrheic keratosis cell cluster test scores 10, 10, 10, and 9 ("successful experiments"). In contrast, Sabouraud cultures with *trichophyton interdigitale* und *klebsiella pneumoniae* resulted in seborrheic keratosis cell cluster test scores 0 and 0 ("failed experiments"). Supernatants of seborrheic keratoses lysed successfully were then compared to failed tests by mass spectrometry as described previously (Schiller-HB et al. Am J Resp and Crit Care Med 2017, 196(10):1298; Simabuco-FM et. al. Mol Cancer 2014, 13:24). Shortly, the mass spectrometry system consisted of a Dionex 3000 Ultimate nano-LC system (Dionex) interfaced to an LTQ Orbitrap Velos mass spectrometer (Thermo Scientific), which was equipped with a nano-ESI source. Protein of culture supernatants was precipitated with trichloroacetic acid (TCA), washed with acetone, and pellets of protein were resuspended with 50µl 8mol/l urea. Protein concentrations were measured by Bradford assay in comparison to bovine serum albumin (BSA). Similar amounts of protein specimens were subjected to proteolytic digest with trypsin following reduction with TCEP and alkylation with iodoacetamide. Resulting peptides were nanoHPLC-ESI-MS/MS. Nominally 400ng peptides were separated on a C18 Column, 0.075x500 mm (Dr. Maisch) using an acetonitrile/0.1% formic acid gradient from 10% to 35% within 100 minutes with 0.5µl/min at 50°C. MS/MS spectra of multiply charged ions were recorded data-dependently. MS/MS data was first searched with Mascot, then database search and label-free quantification was performed with MaxQuant against human sequences and genomic data of bacteria and fungi identical or homologous to expected species. In results, 25 proteins with highest LFQ (label free quantitation) differences were specified in successful experiments, among them keratin 1 (keratin, type II, cytoskeletal 1) and keratin 10 (keratin, type I, cytoskeletal 10). Retrieving these seborrheic keratoses target proteins in the supernatants, proteomics confirms successful breakage of seborrheic keratoses. Among the proteins with highest LFQ intensities in successful experiments, serralysin family metallprotease and hydrolase (WP_012274792.1) revealed high protein expression and are therefore possible protease candidates for seborrheic keratoses.

### Example 4. Fungi-culture supernatant tests, experimental setup and results

Ten seborrheic keratoses were divided in two halves and incubated in 24 well plates with Sabouraud broth liquid bouillon supplemented with antibiotics gentamycin 50ug/ml and chloramphenicol 500ug/ml for 144 hours by room temperature, one half cultured with fungus, the other half cultured without fungus. Fungi were specified by 18s ITS sequencing (institute for microbiology, university of Würzburg, Germany, belonging to Nationales Referenzzentrum für invasive Pilzinfektion NRZMyk). Thereafter pressed between two glasses by 1 kilogram for 16 hours, area of halves incubated with fungi were compared to area of halves incubated in bouillon without fungi. Five experiments resulted in significantly increasing areas ("successful experiments"), resulting in lytic seborrheic keratosis cell cluster test scores 6 (marquandomyces), 10 (aspergillus), 5 (geotrichum), 10 (geotrichum), and 7 (stereum). Five experiments resulted in static areas ("failed experiments"), resulting in lytic seborrheic keratosis cell cluster test scores 0 (geotrichum), 0 (galactomyces), 0 (geotrichum), 0 (castanedomyces), and 0 (aphanoascus). Supernatants of seborrheic keratoses lysed successfully were then compared to failed tests by mass spectrometry as described previously (Schiller-HB et al. Am J Resp and Crit Care Med 2017, 196(10):1298; Simabuco-FM et. al. Mol Cancer 2014, 13:24). Shortly, the mass spectrometry system consisted of a Dionex 3000 Ultimate nano-LC system (Dionex) interfaced to an LTQ Orbitrap Velos mass spectrometer (Thermo Scientific), which was equipped with a nano-ESI source. Protein of culture supernatants was precipitated with trichloroacetic acid (TCA), washed with acetone, and pellets of protein were resuspended with 50µ! 8mol/l urea. Protein concentrations were measured by bradford assay in comparison to bovine serum albumin (BSA).

Similar amounts of protein specimens were subjected to proteolytic digest with trypsin following reduction with TCEP and alkylation with iodoacetamide. Resulting peptides were nanoHPLC-ESI-MS/MS. Nominally 400ng peptides were separated on a C18 Column, 0.075x500 mm (Dr. Maisch) using an acetonitrile/0.1% formic acid gradient from 10% to 35% within 100 minutes with 0.5µl/min at 50°C. MS/MS spectra of multiply charged ions were recorded data-dependently. Mass spectrometry data was first searched with Mascot, then database search and label-free quantification was performed with MaxQuant against human sequences and genomic data of fungi identical or homologous to expected species. Default settings were used for further parameters. Protein identification results were loaded into the Perseus software (version 1.6.14.0). Two protein groups were used for statistical analysis. After grouping und log2 transformation of the LFQ intensities, protein groups were filtered to contain at least five (three) valid values in at least one group. Furthermore, the analysis was carried out involving gauss imputation (to avoid incomplete statistical analysis resulting from zero-point LFQ values), or 0 imputation. Missing values in respective samples were imputed from a normal distribution with a width of 0.3 and a downshift of 1.5 based on the assumption of missing values being a result of the detection limit of the mass spectrometer used and not missing randomly. For the statistical comparison of the groups s0 = 0.1, FDR 0.5 was used.

In results, supernatants of successful experiment, where seborrheic keratoses were lysed, exhibited 80 significantly augmented protein intensities in LFQ (label free quantification), among them keratin 1 (keratin, type II cytoskeletal 1) and keratin 10 (keratin, type I, cytoskeletal 10), the target proteins to lyse seborrheic keratoses. In conclusion, proteomics of supernatants confirmed breakage of seborrheic keratoses.

### Example 5: Formulation of a skin cream comprising microbial supernatant or lysate, step-by-step protocol.

The formulation of a skin cream that comprises a microbial supernatant or lysate requires careful attention to the selection of the microbial strain, the efficacy of the supernatant, and the safety and stability of the final product.
1. Identify the microbial strain: Choose a microbial strain which is positive in ELISA keratin 1 (KRT-1) and/or keratin 10 (KRT-10)-lysis test and/or in lytic seborrheic keratosis cell cluster test; in one embodiment an organism of the family *pseudomonadaceae,* in one embodiment of the genera *pseudomonas* and/or *delftia;* an organism of the family *trichocomaceae,* in one embodiment of the genus *aspergillus;* an organism of the family *dipodascaceae,* in one embodiment of the genus *geotrichum;* an organism of the family *stereaceae,* in one embodiment of the genus *stereum,* and/or an organism of the family *trichocomaceae,* in one embodiment of the genus *paecilomyces,* in one embodiment of the species *P. marquandii* and/or an organism which is positive in a lytic seborrheic keratosis cell cluster test and/or any combination thereof.
2. Culture the microbial strain: Grow the microbial strain in a nutrient-rich medium under controlled conditions, such as temperature and pH.
3. Collect the supernatant: After the microbial culture reaches the stationary phase, centrifuge it to collect the supernatant or lysate, which contains the microbial metabolites, including the active compounds that have the potential to lysis seborrheic keratoses.
4. Test the supernatant/lysate for efficacy: Before formulating the skin cream, test the supernatant/lysate by ELISA keratin 1 (KRT-1) and/or keratin 10 (KRT-10)-lysis test and/or in lytic seborrheic keratosis cell cluster test to confirm the efficacy of the supernatant in lysis seborrheic keratoses.
5. Formulate the skin cream: Select a base cream that is appropriate for the skin type, and add the microbial supernatant/lysate at the appropriate concentration.
6. Mix the cream thoroughly: Mix the cream and the supernatant thoroughly to ensure that the active compounds are distributed evenly throughout the cream.
7. Test the cream for stability: Test the formulated cream for stability by measuring its pH, viscosity, and microbial contamination over time.
8. Test the cream for safety: Perform tests to ensure that the cream is safe for use on human skin. These tests may include irritation tests, allergy tests, and patch tests.
9. Package and label the cream: Once the cream has passed all safety and stability tests, package it in a suitable container and label it with the ingredients, usage instructions, and other relevant information.
10. Store the cream properly: Store the cream in a cool, dry place away from direct sunlight to maintain its stability and efficacy.

Preparation of further topical compositions like for a solution or a patch or a facemask would be performed under analog procedures.

## Claims

1. A topical composition comprising a therapeutically effective amount of an active agent which suppresses keratin 1 (KRT 1) and/or keratin 10 (KRT 10) activity for use in the treatment of seborrheic keratosis.

2. The use according to claim 1, wherein the active agent is selected from a group consisting of a siRNA specific for keratin 1 (KRT1) and/or keratin 10 (KRT 10), an antigen-binding compound specific for keratin 1 (KRT1) and/or keratin 10 (KRT 10), and a protease which degradates keratin 1 (KRT1) and/or keratin 10 (KRT 10), as well as any combination thereof.

3. The use according to any of claims 1 or 2, wherein the active agent is selected from the group consisting of a calpain; a cathepsin L; a matrix metalloprotease (MMP), such as MMP-1 and/or MMP-9; a kallikrein, such as kallikrein-5 and/or kallikrein-7; a serine protease, such as elastase; a cysteine protease, such as papain; a lysosomal aspartic protease, such as cathepsin D; a caspase, such as caspase-14; a KRT1-siRNA; Jagged1; N-[N-(3,5-Difluorphenacetyl)-L-alanyl]-S-phenylglycin-tert-butylester (DAPT); a serralysin family protease, such as from *Pseudomonas panacis;* and a hydrolase, such as from *Pseudomonas spec,* as well as any combination thereof.

4. The use of the topical composition according to any of the previous claims, wherein the active agent is and/or is derived from a supernatant of a culture of a microbial or fungal organism and/or a supernatant of a cell lysate of a culture of a microbial or fungal organism and wherein the active agent passed the KRT1/KRT10-lysis test and/or lytic seborrheic keratosis cell cluster test with a score of at least 3.

5. A topical composition comprising a therapeutically effective amount of an active agent which degradates keratin 1 (KRT1) and/or keratin 10 (KRT 10).

6. The topical composition according to claim 5, wherein the topical composition comprises a supernatant of a culture of a microbial or fungal organism and/or a supernatant of a cell lysate of a culture of a microbial or fungal organism, comprising an active agent which degradates keratin 1 (KRT1) and/or keratin 10 (KRT 10).

7. The topical composition according to any of claims 5 or 6, wherein the active agent is selected from the group consisting of a calpain; a cathepsin L; a matrix metalloprotease (MMP), such as MMP-1 and/or MMP-9; a kallikrein, such as kallikrein-5 and/or kallikrein-7; a serine protease, such as elastase; a cysteine protease, such as papain; a lysosomal aspartic protease, such as cathepsin D; a caspase, such as caspase-14; a KRT1-siRNA; Jagged1; N-[N-(3,5-Difluorphenacetyl)-L-alanyl]-S-phenylglycin-tert-butylester (DAPT); a serralysin family protease, such as from *Pseudomonas panacis;* and a hydrolase, such as from *Pseudomonas spec,* as well as any combination thereof.

8. The topical composition according to claim 6, wherein the microbial or fungal organism is selected from a group consisting an organism of the family *pseudomonadaceae,* such as of the genera *pseudomonas* and/or *delftia;* an organism of the family *trichocomaceae,* such as of the genus *aspergillus;* an organism of the family *dipodascaceae,* such as of the genus *geotrichum;* an organism of the family *stereaceae,* such as of the genus *stereum,* and/or an organism of the family *trichocomaceae,* such as of the genus *paecilomyces,* such as of the species *P. marquandii;* and/or any combination thereof.

9. The topical composition according to claim 8, wherein the microbial or fungal organism is disclosed in the *"2023 list of QPS-recommended micro-organisms for safety risk assessments carried out by EFSA"* (version 15; as of January 25, 2023; version-ID: 10.5281/ze-nodo.7554079).

10. The topical composition according to any of claims 6 to 9, wherein supernatant of a culture of a microbial or fungal organism and/or a supernatant of a cell lysate of a culture of a microbial or fungal organism passed the KRT1/KRT10-lysis test and/or lytic seborrheic keratosis cell cluster test with a score of at least 3.

11. The topical composition according to any of claims 5 to 10, formulated as an ointment, a cream, a cream ointment, a gel, a hydrogel, a lotion, a solution, a fatty ointment, a powder, and/or a paste.

12. The topical composition according to any of claims 5 to 11, and further comprising at least one compound selected from the group consisting of DMSO, salicylic acid, glycolic acid, urea, glycerin, hyaluronic acid, aloe vera, panthenol, jojoba oil, almond oil, argan oil, coconut oil, vitamins such as vitamin E or vitamin C, shea butter, lactic acid, fruit acid, chamomile, allantoin, or bisabolol, collagen, elastin, antifungal and antibacterial agents such as miconazole, clotrimazole, triclosane, zinc oxide; anti-inflammatory agents such as calamine or hydrocortisone; DTT; skin protectants, such as zinc oxide or petrolatum; exfoliants such as baking soda or kaolin clay; and sunscreen ingredients such as zinc oxide or titanium dioxide, as well as any combination thereof.

13. A skin patch and/or facemask for the use according to any of claims 1 to 4 and/or containing the topical composition according to any of claims 5 to 12.

14. A method for producing the topical composition according to any of claims 5 to 13, comprising the steps of
a. culturing an organism selected from a group consisting an organism of the family *pseudomonadaceae,* such as of the genera *pseudomonas* and/or *delftia;* an organism of the family *trichocomaceae,* such as of the genus *aspergillus;* an organism of the family *dipodascaceae,* such as of the genus *geotrichum;* an organism of the family *stereaceae,* such as of the genus *stereum,* and/or an organism of the family *trichocomaceae,* such as of the genus *paecilomyces,* such as of the species *P. marquandii* and/or an organism; and/or any combination thereof;
b. collecting a supernatant of the culture and/or a supernatant of a cell lysate of the culture;
c. optionally, concentrating and/or purifying the supernatant of step b.;
d. subjecting the supernatant of the culture and/or a supernatant of a cell lysate to a KRT1 /KRT10-lysis test and/or lytic seborrheic keratosis cell cluster test and progressing only with a supernatant of the culture and/or a supernatant of a cell lysate which passed the KRT1/KRT10-lysis test and/or lytic seborrheic keratosis cell cluster test with a score of at least 3;
e. formulating the supernatant into a topical composition selected from the group consisting of an ointment, a cream, a cream ointment, a gel, a hydrogel, a lotion, a solution, a fatty ointment, a powder, and/or a paste;
f. optionally, adding further at least one compound selected from the group consisting of DMSO, salicylic acid, glycolic acid, urea, glycerin, hyaluronic acid, aloe vera, panthenol, jojoba oil, almond oil, argan oil, coconut oil, vitamins such as vitamin E or vitamin C, shea butter, lactic acid, fruit acid, chamomile, allantoin, or bisabolol, collagen, elastin, antifungal and antibacterial agents such as miconazole, clotrimazole, triclosane, zinc oxide; anti-inflammatory agents such as calamine or hydrocortisone; DTT; skin protectants, such as zinc oxide or petrolatum; exfoliants such as baking soda or kaolin clay; and sunscreen ingredients such as zinc oxide or titanium dioxide, as well as any combination thereof;
g. thereby receiving a topical composition for the treatment of seborrheic keratosis.

15. A kit for the use according to any of claims 1 to 4, comprising the topical composition according to any of claims 5 to 12, and/or the skin patch and/or facemask according to claim 13, and/or the topical composition received by the method of claim 14, further comprising at least one other item selected from an instruction leaflet, a protection device and an applicator, as well as any combination thereof.
